# NEW EUROPEAN PATENT SPECIFICATION

(11) **EP 1 587 514 B3**
(45) Date of publication of this specification: **22.06.2011**
(45) Mention of the grant of the patent: 14.06.2006
(21) Application number: 04702472.4
(22) Date of filing: 15.01.2004
(51) Int. Cl.: A61K 31/4468

(54) **A RAPID-ACTING PHARMACEUTICAL COMPOSITION**
SCHNELL WIRKENDE PHARMAZEUTISCHE ZUSAMMENSETZUNG
COMPOSITION PHARMACEUTIQUE A EFFET RAPIDE

(30) Priority: 31.01.2003 US 443857 P
(43) Date of publication of application: 26.10.2005
(73) Proprietor: Orexo AB, 751 05 Uppsala (SE)
(72) Inventor: NYSTRÖM, Christer, S-756 61 Uppsala (SE); BREDENBERG, Susanne, S-113 50 Stockholm (SE)
(74) Representative: McNeeney, Stephen Phillip
(86) International application number: PCT/SE2004/000037
(87) International publication number: WO 2004/067004

(56) References cited:
- EP-A2- 0 681 833
- WO-A1-00/16750
- BREDENBERG S. ET AL: 'In vitro and in vivo evaluation of a new sublingual tablet system for rapid oromucosal absorption using fentanyl citrate as the active substance' EUROPEAN JOURNAL OF PHARMACEUTICAL SCIENCES vol. 20, 2003, pages 327 - 334, XP002980244
- BREDENBERG S. ET AL: 'In-vitro evaluation of bioadhesion in particulate systems and possible improvement using interactive mixtures' JOURNAL OF PHARMACY AND PHARMACOLOGY vol. 55, 2003, pages 169 - 177, XP002980245

## Description

### Field of the invention

The present invention relates to a rapidly acting pharmaceutical composition for sublingual or intranasal administration of a pharmaceutical agent, to a method for preparing such acomposition, and to the use of such a composition for the manufacture of a medicament for the treatment of acute disorders.

### Background of the invention

Acute and/or severe disorders are a common cause of emergency treatment or hospitalization. One of the most common disorders of this type is acute or breakthrough pain. In cancer patients, pain is usually treated with non-steroid anti-inflammatory drugs (NSAIDs) and opiates alone or in combination. Opioid-requiring cancer pain patients are usually given slow-release opiates (slow-release morphine or ketobemidone or transdermal fentanyl). A characteristic feature of cancer pain are periods of inadequate analgesia (breakthrough pain) Most often they are due to increased physical activity of the patient. However, treatment of breakthrough pain by administration of increased time contingent doses of long-acting analgesics causes adverse side effects such an excess sedation, nausea, and constipation.

Other disorders and conditions which require a fast-acting treatment are, for example, pulmonary edema, gastroesophageal reflux, insomnia and nephrolitiasis.

Presently available oral, rectal, intranasal or sublingual formulations have relatively lengthy onset times or erratic absorption characteristics that are not well suited to control acute disorders.

Conditions of acute operative/postoperative or traumatic/ posttraumatic pain as well as pain due to severe disease (e.g. myocardial infarction, nephrolithiasis, etc.) is usually treated with opioid analgesics which are administered parenterally (by intravenous or intramuscular administration) to obtain a rapid onset of analgesia. In such cases, rapid-onset oral alternatives are of considerable therapeutic interest. Also for the treatment of other acute disorders, it is of considerable interestto provide fast-acting therapeutic compositions which may be administered orally orby the intranasal route instead of parenterally or rectally.

However, many pharmaceutically active agents which would be advantageous to adminster orally are not suitable to be swallowed. They may, for example, be inactivated by the gastro-intestinal liquids, have a slow action because of a low solubility in the aqueous medium, or be highly susceptible to metabolism by gastro-intestinal enzymes and have poor absorptiom properties, as exemplified for peptide hormones. It is therefore more preferable to arrange for the active component to be taken up through the mucous membranes of the oral or nasal cavity. For the oral cavity, the most preferred way of administration is via the sublingual route. In this administration, a dosage unit of the pharmaceutical composition is placed under the tongue, and the active component is absorbed through the surrounding mucous membranes. However, with this way of administration, the risk that the patient swallows the medication by swallowing saliva is well known.

For the treatment of acute pain may be used fentanyl, N-(1-phenethyl-4-piperidyl)-propioanilide, or one of its pharmaceutically acceptable salts. This compound is an opioid agonist and shares many of the pharmacodynamic effects of opiates such as morphine and meperidine. However, compared to these opiates, fentanyl exhibits little hypnotic activity, rarely induces histamine release, and respiratory depression is more short-lived. Fentanyl is commercially available for intravenous, intrabucchal (lozenge-transmucosal) and transdermal administration.

Following parenteral administration of fentanyl, the analgesic action is more prompt and less prolonged than that of morphine and meperidine. The onset of analgesia following i.v. administration is rapid. Peak analgesia is obtained within a few minutes. Following transbucchal administration by a lozenge, consumption of the lozenge is usually complete within 30 min and peak plasma concentrations appear after around 20 minutes, as described by e.g. Farrar et al., J. Natl. Cancer Inst., 1998, 90(8), p. 611-616. Analgesia is apparent within 5-15 min and peaks at about 20-50 min. While this is an improvement over oral administration for gastrointestinal uptake, a quicker onset of analgesia would be of substantial benefit to the patient. In addition, substantial amounts of lozenge-administered fentanyl are swallowed by the patient. This is not desirable and results in the administration of excessive amounts of the drug, which may give rise to side effects.

### Objects of the invention

It is one object of the invention to provide for the treatment of acute disorders by peroral or intranasal administration of-at least one pharmaceutically active agent in a manner giving rise to pharmacologically effective plasma levels of said agent or agents within a short time after administration.

It is another object of the invention to provide a pharmaceutical composition suitable for that purpose.

It is a further object of the invention to provide a method of making such a composition.

It is an additional object of the invention to provide a use of a medicament for sublingual or intranasal administration containing a physiologically effective dose of at least one pharmaceutically active compound for the manufacture of a medicament for the treatment of acute disorders.

### Description of the drawing

The sole figure of the drawing shows the result of a test of the bioadhesive strength of a composition according to the invention. It is a diagram showing the maximum tensile strength against the concentration.

### Summary of the invention

According to the invention, the peroral treatment of acute disorders comprises sublingual administration of an ordered mixture comprising a pharmacologically effective amount of at least one pharmaceutically active agent Said agent or agents is administered sublingually in combination with a bioadhesion and/or mucoadhesion promoting compound. In the same manner, the same composition is also useful for intranasal administration.

Further according to the invention, there is also provided a single-dose pharmaceutical composition for sub-lingual or intranasal administration, comprising a pharmacologically effective amount of at least one pharmaceutical active agent. Said composition also contains a bioadhesion or mucoadhesion promoting compound. This composition reduces erratic drug absorption via swallowed saliva and enables the administration of small amounts of said agent or agents. Therefore, it substantially reduces the risk of side effects and intrapatient as well as interpatient variation of therapeutic response. Thereby the risk of drug accumulation is reduced, making the pharmaceutical preparation well suited for repeated dosing in patients suffering-from acute disorders.

The amount of active agent or agents contained in the pharmaceutical composition of the invention is obviously dependent on a number of factors, which are to be evaluated by the treating physician. Among such factors may be mentioned the specific agent used and the type of disorder being treated, the medical status of the patient, and others.

When fentanyl is used for the treatment of acute or breakthrough pain, the composition of the invention should contain from 0.05 up to 20 weight percent of fentanyl or one of its pharmaceutically acceptable salts. More preferably, the compositions contains from 0.05 to 5 weight percent of fentanyl, and especially from 0.1 to 1 weight percent. The contents can also be expressed as the amount of fentanyl in a dose unit of the composition, such as a tablet. In this case, a dose unit should contain from 0.025 to 10 mg, and preferably 0.05 to 2 mg of fentanyl. When the fentanyl is used in the form of a salt, these percentages and amounts should be recalculated accordingly.

Still further according to the invention, the sublingual or intranasal composition comprises an ordered mixture of one or more bioadhesive and/or mucoadhesive carrier substances coated with the pharmaceutically active agent or agents in a fine particulate form. According to the invention, the carrier substance or substances are insoluble or sparingly soluble in water and is defined hereinafter. The term "ordered mixture" is meant to denote the use of a fine particulate quality of active ingredient (s) intimately mixed with coarser excipient particles. Then, the fine drug particles are attached essentially as primary particles on the surface of the excipient (carrier) particles. Also terms like "interactive mixture" or "adhesive mixture" can be used interchangeably, in this context.

It is preferred to formulate the composition according to the invention by use of a variant of the technology for formulating rapidly dissolving ordered-mixture compositions disclosed in European patent EP 0 324 725. In these compositions, the drug in a finely dispersed state covers the surface of substantially larger, water-soluble carrier particles. Such compositions disintegrate rapidly in water, thereby dispersing their contents of microscopic drug particles.

The dissolution of a fine particulate drug from ordered mixtures has hitherto been associated with the use of soluble carriers. This approach is characterised by a rapid dissolution of the carrier, thus quickly liberating the fine drug particles. These drug particles, now presented as discrete units, will rapidly dissolve, due to favourable hydrodynamics. This approach has previously been limited to the use of large volumes of dissolving fluid. It has been understood that it is only when drug particles are liberated to larger volumes of dissolving liquid that the dissolution is not hindered by saturation phenomenon or unfavourable hydrodynamics.

However, in the published PCT application No. WO 00/16750, the use of ordered mixtures with soluble carriers has been applied to sub-lingual administration. In spite of the limited volume of dissolving fluid (saliva) in the oral cavity it was found that a rapid dissolution and subsequent drug uptake could be achieved. It has now, unexpectedly, been realised that also insoluble or sparingly soluble carriers can be used with the same result. It is believed that the optimal exposure of discrete drug particles. (i.e, in a non-agglomerated form) on the surface of the coarser carrier particles represents a determining factor for the rapid dissolution. Since the drug is positioned on the surface of the main tablet component, the large surface area taking part in dissolution will give a rapid dissolution in spite of the fact that these drug particles are not liberated from the insoluble carrier, prior to dissolution. Thus, dissolution can rapidly take place also from drug particles attached to a carrier, as long as the drug is in very fine particulate form and present as discrete, non-agglomerated units. Another prerequisite is that the drug is used in low proportions. Preferably the dose should be lower than 10 mg and more preferably lower than 2 mg.

An advantage with insoluble carriers over soluble carriers is their improved tendency to adhere to the mucosa after being coated with a finer, bio/muco-adhesive component. It was found that a soluble carrier, will soon after administration, start to dissolve and thereby the mucoadhesion will decrease. An insoluble carrier coated with bioadhesive particles, on the other hand, will remain attached to the mucosa for a longer time and an improved mucoadhesion will result This is further explained in Example 1.

A bioadhesion and/or mucoadhesion promoting agent is additionally added to the carrier particles according to the invention. The bioadhesion and/or mucoadhesion promoting agent is effective in making the active agent or agents adhere to the oral or nasal mucosa and may, in addition, possess properties to swell and expand in contact with waater. The bio/mucoadhesion promoting agent must then be present on the surface of the carrier particles.

The expression "mucoadhesion" is meant to denote an adhesion to mucous membranes which are covered by mucus, such as those in the oral cavity, while the expression "bioadhesion" is meant to denote an adhesion to biological surfaces more in general, including mucous membranes which are not covered by mucus. These expressions generally overlap as definitions, and may usually be used interchangeably, although the expression "bioadhesive" has a somwhat wider scope. In the present specification and claims, the two expressions serve the same purpose as regards the objects of the invention, and this has been expressed by the use of the common term "bio/mucoadhesion".

Suitably the carrier particles contain from 0.1 up to 40 weight percent of bio/mucoadhesion promoting compound, based on the total composition, In practice, contents below 1 weight percent have been found to give an insufficient bio/ mucoadhesive effect. The preferred range of bio/mucoadhesion promoting agent content is from 2 to 25 weight percent

It is preferred that the bio/mucoadhesion promoting agent is a polymeric substance, preferably a substance with an average molecular weight above 5,000 (weight average) . The level of hydration of the mucosa adhesion promoting agent interface is of importance in the development of bio/mucoadhesive forces. Therefore, the faster the swelling of the polymer, the faster is the initiation of bio/mucoadhesion. The hydration of bioadhesive compounds also makes them useful as absorption enhancers according to the invention.

Preferably, the carrier particle size is less than 750 µm, and more preferably from 50 to 500 µm. Although particle sizes outside the indicated range can be used, practical difficulties are experienced when formulating pharmaceutical preparations from particles having such sizes. The carrier used may comprise any substance which is pharmaceutically acceptable, is insoluble or sparingly soluble in water, and which can be formulated into particles fit for incorporating a bio/mucoadhesion promoting agent. A number of such substances are known to the person skilled in this art. As suitable examples may be mentioned polymers such as celluloses (e.g. microcrystalline cellulose), cellulose derivatives, starch, starch derivatives, cross-linked polymers based on e.g. starch, cellulose and polyvinylpyrrolidone. Furthermore, inorganic salts can be used, such as dicalcium phosphate dihydrate. Mixtures or co-processed qualities of the above-mentioned materials may also be used.

In accordance with one particularly preferred aspect of the invention, the carrier also possesses fragmenting behaviour. By fragmentation behaviour is meant that the carrier is to some extent a brittle material which is readily crushed or broken up when a pharmaceutical composition of which it forms a part is compacted into tablets. This effect is especially pronounced when the bio/mucoadhesion promoting agent also serves as a disintegrant. Dicalcium phosphates have been found to be particularly suitable as fragmentation promoting agents.

The addition of a pharmaceutically acceptable surfactant to the composition is also a preferred feature of the invention. The increased wetting effect of the surfactant enhances the wetting of the carrier particles, which results in faster initiation of the bio/mucoadhesion. The surfactant should be in a finely dispersed form and intimately mixed with the active agent or agents. The amount of surfactant should be from 0.5 to 5 weight percent of the composition, and preferably then from 0.5 to 3 weight percent.

As examples of suitable surfactants may be mentioned sodium lauryl sulfate, polysorbates, bile acid salts and mixtures of these.

A variety of polymers known in the art can be used as bio/mucoadhesion promoting agents. In addition to their polymeric nature, their ability to swell is important On the other hand, it is also important that they are substantially insoluble in water. Their swelling factor by volume when brought into contact with water or saliva should preferably be at least 10, while a factor of at least 20 is more preferred. Examples of such bio/mucoadhesion promoting agents Include cellulose derivatives such as hydroxypropylmethyl cellulose (HPMC), hydroxyethyl cellulose (HEC), hydroxypropyl cellulose (HPC), methyl cellulose, ethyl hydroxyethyl cellulose, carboxymethyl cellulose and sodium carboxymethyl cellulose (NaCMC); starch derivatives such as moderately cross-linked starch; acrylic polymers such as carbomer and its derivatives (Polycarbophyl, Carbopol® , etc.); polyethylene oxide (PEO); chitosan (poly- (D-glucosamine)); natural polymers such as gelatin, sodium alginate, pectin; scleroglucan; xanthan gum; guar gum; poly co-(methylvinyl ether/maleic anhydride); and crosscaramellose. Combinations of two or more bio/mucoadhesive polymers can also he used. More generally, any physiologically acceptable agent showing bio/mucoadhesive characteristics may be used successfully to be incorporated in the carrier. Bio/mucoadhesiveness can be determined in vitro, e.g. according to G. Sala et al., Proceed. Int. Symp. Contr. Release. Bioact. Mat. 16:420, 1989.

Some suitable commercial sources for representative biolmucoadhesive polymers include:
Carbopol® acrylic copolymer - BF Goodrich Chemical Co, Cleveland, 08, USA;
HPMC - Dow Chemical Co., Midland,), MI, USA;
NEC (Natrosol) - Hercules Inc., Wilmington, DE., USA;
HPC (Klucel®) - Dow Chemical Co., Midland, MI, USA;
NaCMC - Hercules Inc. Wilmington, DE.. USA;
PEO - Aldrich Chemicals, USA;
Sodium Alginate, - Edward Mandell Co., Inc., Carmel, NY, USAi
Pectin - BF Goodrich Chemical Co., Cleveland, OH, USA.'
Ac-Di-Sol® (modified cellulose gum with a high swellability) - FMC Corp., USA;
Actigum, - Mero-Rousselot-Satia, Baupte, France;
Satiaxane - Sanofi Bioindustries, Paris, France;
Gantrez® - ISP, Milan, Italy;
Chitosan - Sigma, St Louis, MS, USA;

Depending on the type and the proportion of the bio/mucoadhesion promoting agent used, the rate and intensity of bio/mucoadhesion may be varied. According to one of the preferred aspects of the invention, substances with high and rapid capacity for swelling are preferred.

In order for the pharmaceutical composition of the invention to function properly when a bio/mucoadhesion promoting agent is added thereto, this agent must be positioned at the surfaces of the carrier particles. The bio/mucoadhesion promoting agent can then be admixed to the carrier particles in several ways. In a preferred embodiment of the invention, a fine particulate quality of the bio/mucoadhesion promoting agent is mixed together with the coarse carrier for a sufficient time to produce an ordered mixture, where the finer particles exist as discrete primary particles adhered to the surfaces of the carrier particles. Thus, the bio/mucoadhesion promoting agent is admixed in the same way as the active compound described in European patent No. 0 324 725.

The bio/mucoadhesion promoting agent suitably has a particle size between 1 and 100 µm. When the particles of this agent are to be mixed with the carrier particles to form an ordered mixture, their size lies within the lower part of the size interval, and suitably their size is then below 10 µm.

The invention is particularly directed to the administration of drugs which are used for the treatment of medical conditions where a rapid and transient effect is desirable, such as pain, insomnia, allergic conditions and pulmonary oedema. As non-limiting examples of such drugs may be mentioned morphine (analgetic), fentanyl (analgetic), alfentanyl (analgetic), sufentanyl (analgetic), buprenorphine (analgetic), pizotifen (analgetic), sumatriptan (analgetic), indomethacin (analgetic), sulindac (analgetic), diclofenac (analgetic), ketorolac (analgetic), piroxicam (analgetic), tenoxicam (analgetic), ibuprofen (analgetic), naproxen (analgetic), ketoprofen (analgetic), butazolidine (analgetic), phenylbutazone (analgetic), diazepam (insomnia), oxazepam (insomnia), zopiclone (insomnia), zolpidem (insomnia), propiomazin (insomnia), valeriana (insomnia), levomepromazin (insomnia), cyclizine (allergy), cetirizine (allergy), terfenadine (allergy), acrivastine (allergy); fexofenadine (allergy) and furosemide (diuretic).

Other drugs which benefit from an enhanced absorption and which may be used for medical conditions where a rapid onset of the action is desirable include, without any limiting sense, various peptides and enzymes, such as atrial natriuretic peptides (ANP, ANF, auriculin) (diuretics), brain natriuretic peptides (diuretics), platelet aggregation inhibitors (anticoagulants), streptokinase (anticoagulant), heparin (anticoagulant), urokinase (anticoagulant), renin inhibitors (hypertension), insulin (antidiabetic), and sleep inducing peptide (insomnia).

Further examples of drugs where exposure to gastric acid has to be avoided and where the swallowing of active drug containing saliva can be minimised by means of the bio/mucoadhesive properties of the present formulations include, without any limiting sense, benzimidazole derivatives used as H⁺, K⁺ and ATPase inhibitors (gastric acid reduction), such as omeprazole, pantoprazole, perprazole and lansoprazole. Other H⁺, K⁺ and ATPase inhibitors include alyll isothiocyanate, trifluorperazide, nolinium bromide, RP 40749 and fenoctimine.

The invention is particularly suitable for the administration of fentanyl and its pharmacologically acceptable salts, such as the citrate or maleate, which are not readily soluble in water. The particles of fentanyl or salt thereof will suitably have a maximum particle size of about 24 µm but will preferably not be greater than about 10 µm. Fentanyl is caused to adhere to the carrier particles e.g. by dry mixing of the ingredients during a period of time of sufficient length. This time period can vary according to the mixing equipment used. A person skilled in the art will have no difficulty in determining by experimentation a suitable mixing time for a given combination of active substance, bio/mucoadhesion promoting agent and carrier, by using a particular mixing equipment.

A further preferred aspect of the invention comprises the incorporation of a disintegrating agent in the composition ofthe invention. Such an agent which will accelerate the dispersion of the carrier particles. Examples of disintegrating agents according to the invention include cross-linked polyvinylpyrrolidone, carboxymethyl starch, natural starch, microcrystalline cellulose, cellulose gum and mixtures of these. A preferred content of disintegrating agent is from 1 % to 10 % of the composition. As can be seen, the definitions of the disintegrating agent and the bio/mucoadhesion promoting agent overlap somewhat, and it may be preferred that both functions are served by the same substance. However, it is important to note that these two categories of excipients are not equivalent, and there are efficiently functioning disintegrants which do not possess bio/mucoadhesive properties, and vice versa.

The ordered mixtures prepared in accordance with the present invention can be used as such for intranasal administration. Normally the powder mixture is then insufflated to the nasal cavity by the aid of some type of delivery device. The ordered mixture can also be incorporated into various kinds of pharmaceutical preparations intended for sublingual administration. Irrespective ofthe form given to the preparation, it is important that the preparation is essentially free from water, since its bio/mucoadhesion promoting character results from its practically instantaneous hydration when brought into contact with water or saliva. Premature hydration would drastically decrease the mucoadhesion promoting properties and result in a premature dissolution of the active substance.

A pharmaceutical composition for the preferred sublingual route of administration can be obtained by combining an aforementioned ordered mixture with conventional pharmaceutical additives and excipients used in the art for sub-lingual preparations. Appropriate formulation methods are well known to the person skilled in the art; see, for instance, Pharmaceutical Dosage Forms: Tablets. Volume 1, 2nd Edition, Lieberman H A et al.; Eds.; Marcel Dekker, New York and Basel 1989, p. 354-356, and literature cited therein. Suitable additives comprise additional carrier agents, preservatives, lubricants, gliding agents, disintegrants, flavorings, and dyestuffs.

Thus, the invention provides a dosage form which is easy and inexpensive to manufacture, enables rapid active substance release, promotes a rapid uptake ofthe active agent or agents through the oral or nasal mucosa, and enhances the upptake of otherwise poorly soluble substances, such as peptides.. The use of a low dose of active agent is provided for, supporting a short duration of action while enabling a repeated dosing schedule for patients in need of treatment of recurrent acute disorders.

The invention will now be illustrated in more detail by reference to examples.

### Example 1

### Materials

Dibasic calcium phosphate dihydrate (DCP) (Emcompress, Edward Mendell Co, Inc, USA) with low aqueous solubility and Mannitol (granulated quality, Roquette, France) with high aqueous solubility were used as non-bioadhesive carrier materials in the preparation of ordered mixtures. A size fraction of 180-355 µm for each material was obtained by dry sieving (Retsch, Germany).

Cross-linked carboxymethyl cellulose sodium (Ac-Di-Sol, FMC, Cork, Ireland) were used in a fine divided form to represent a material with mucoadehsive/bioadhesive properties. The fine particle size fraction of Ac-Di-Sol was obtained by milling in a mortar grinder (Retsch, Germany) followed by air classification (100 MZR, Alpine, Germany).

### Primary characterisation of test materials

All powders were stored at 40% RH and room temperature, for at least 48 hours before characterisation and mixing. The external surface area of the coarser size fractions (180- 355 µm) of Mannitol and DCP was determined using Friedrich permeametry (n=3) (Eriksson et al 1990). Blaine permeametry was used to determine the external surface area of the Ac-Di-Sol powder (Alderbom et al 1985) (Table 1).

### Preparation of ordered/interactive mixtures

Milled Ac-Di-Sol (Table 1) was added to Mannitol or DCP (both 180-355 µm) in varying proportions to obtain different concentrations of Ac-Di-Sol. The powders were mixed in glass jars in a 2L Turbula mixer (W.A. Bachofen AG, Basel, Switzerland) at 120 rpm for 24 hours. Mixing was performed in accordance with previous studies (Westerberg 1992; Sundell-Bredenberg and Nyströn 2001) and the mixture homogeneity was visually confirmed.

### Measurements of bioadhesive/mucoadhesive properties

### Materials and characterisation of the mucosa

Fresh pig intestine was collected at a slaughterhouse (Swedish Meat AB, Uppsala, Sweden) and used fresh or was frozen until required. Before use, the frozen intestine was thawed in buffer solution at 4°C overnight. The buffer solution used was Krebs-Ringer Bicarbonate (Sigma-Aldrich Chemie GmbH, Steinheim, Germany) with a pH of 7.4.

To test the quality of the mucus layer and the effect of handling the mucosa, several tissue specimens were stained with Alcain blue, partly according to the method by Come et al I (1974). Both fresh and frozen tissues were then soaked for two hours in TRIS (TRIZMAHydrochloride, Sigma-Aldrich Chemie GmbH, Steinheim, Germany) buffered sucrose solution (Sigma-Aldrich Chemie GmbH, Steinheim, Germany) with Alcian blue 8 GX, (Certistain, Merck, Germany) (1 mg/ml). The tissues were rinsed in TRIS/sucrose buffer and visually studied. On evaluating the quality of the mucus layer and the effect of handling the tissue, it was noted that neither the thawing process (in buffer solution at 4 °C) nor handling affected the quality of the mucosa, i.e. the mucus layer remained intact, and therefore both fresh and frozen mucosa were used in this study.

### Adhesion test

A TA-HDi texture analyser (Stable Micro Systems, Haslemere, UK) with a 5 kg load cell and associated software was used for the bioadhesion studies. The pig intestine was cut into approximately 2 cm² pieces and placed in a tissue holder. The powder mixtures [using double-sided tape (Scotch, 3M Svenska AB, Sollentuna, Sweden)] was attached to the upper probe The application of the powder mixtures was performed by immersing the probe in to a powder bed and there after the probe was gently shaken to remove any excess, in order to achieve a monolayer of particles, which was visually validated. After spreading 30 µl of buffer with a pipette on the mucosa to standardise hydration, the studied material was brought into contact with the mucosa under a force of 0.5 N over 30 seconds. The probe was then raised at a constant speed of 0.1 mm/s and the detachment force was recorded as a function of displacement. The detachment force was measured at a sampling rate of 25 measurements/second throughout the measuring cycle. The maximum force monitored, i.e. the fracture force, was determined using the computer software Texture Expert Exceed (Stable Microsystems, Haslemere, UK).The tensile stress (N/cm²) was obtained by dividing the detachment force by the area of the probe.

### Results regarding the useof ordered/interactive mixtures (the addition of fine bioadhesive particles) to increase the bioadhesive properties of a carrier material

### The effect of the amount of bioadhesive/mucoadhesive component

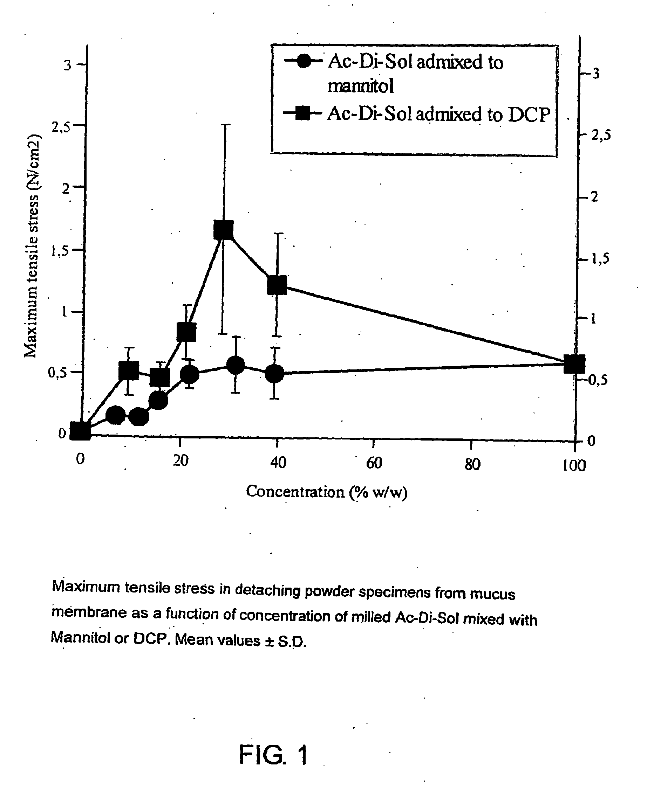

Tensile stress between the mucosa and the non-bioadhesive carrier particles were improved (p<0.0001) when the coarse DCP or Mannitol was mixed with the fine particle size of Ac-Di-Sol (Fig. 1). The bioadhesive properties improved (p<0.05) initially with increases in the concentration of Ac-Di-Sol.

Ordered mixtures of DCP containing the two highest concentrations of Ac-Di-Sol (28.2 and 39.3% w/w) gave values for tensile stress significantly higher (p<0.05) than for powders of pure Ac-Di-Sol (Fg. 1). This effect was however not seen (p>0.1) with mixtures containing Mannitol, probably because of the higher water solubility of Mannitol, as discussed below. As seen in Fig. 1, the increase in bioadhesive strength is significant (p<0.01) up to a certain amount of added Ac-Di-Sol. When the amount exceeded approximately 20%w/w, the significant increase (p>0.1)in tensile stress.

### The effect of carrier solubility

DCP mixtures were significantly more (p<0.02) bioadhesive (had higher tensile stress than Mannitol mixtures). This may be a result of the higher water solubility of Mannitol. Thus, the fracture for the Mannitol mixtures might have gone through dissolved peripheral regions of the interactive mixtures and not entirety through the mucus layer.

**Table 1. Primary characteristics of test materials. Mean values (± s.d.).**

| Material | Particle size fraction (µm) | Apparent particle density (g/cm³)^{a} | External specific surface area (cm²/g)^{b} |
|---|---|---|---|
| Ac-Di-Sol | Milled | 1.607 (±0.001) | 6400 (±91), 6700 (±180) |
| Mannitol | 180-355 | 1.486 (±0.000) | 290 (±6.5) |
| DCP | 180-355 | 2.884 (±0.001) | 440 (±3.7) |
| ^{a} Measured with a helium pycnometer (AccuPyc 1330 Pycnometer, Micromeritics, USA) (n=3). | | | |
| ^{b} Measured with a Friedrich permeameter (Eriksson et al 1990) or Blaine permeameter (Alderborn et al 1985) (n=3). | | | |

### Conclusions

The tensile stress between the mucosa and the coarser Mannitol or DCP powders were improved (p<0.0001) when these were mixed with the fine particulate Ac-Di-Sol. This indicates that addition of materials with a higher adhesion tendency will increase the adhesion of another, less bioadhesive material, such as the carrier materials.
The use of interactive mixtures of bioadhesive powders with aqueous-insoluble carriers rather than with aqueous-soluble carriers is unexpectedly superior, especially at a proportion close to monoparticulate surface coverage.
Thus, it is concluded that such interactive mixtures, using sparingly soluble carriers, is an interesting formulation tool in the development of bioadhesive formulations such as instant release formulations for sublingual administration.

### Example 2. Preparation of a rapidly disintegrating tablet with bio/mucoadhesion promoting properties.

A batch of 1000 tablets was produced from the following compositions: 82.5 g of dibasic calcium phosphare dihydrate (DCP) having a particle size from about 250 to 450 microns, was mixed with 500 mg of micronized fentanyl over a period of 50 hours. The resulting mixture was admixed with 10.0 g micronised sodium alginate (bio/mucoadhesion promoting agent) over a period of 5 hours. Thereafter, 5.0 g of Avicel® Ph 101 (acting as binder) and 2.0 g of Ac-Di-Sol® (modified cellulose gum acting as effective disintegrant) was admixed for 60 minutes. The resulting mixture was mixed with 0.5 g magnesium stearate (lubricant) for 2 minutes and the final tablet mass was then compacted into tablets at a compaction pressure of 200 MPa, each tablet having a weight of 100 mg and containing 0.5 mg of fentanyl.

### Example 3. Preparation of rapidly disintegrating tablets for the administration of atrial natriuretic peptide (ANP)

Rapidly disintegrating tablets with bio/mucoadhesive properties which in addition enhance absorption of large molecules in sublingual administration were prepared according to Example 2, each tablet containing 0.7 mg ANP. However, in this composition the sodium alginate was removed and the addition of Ac-Di-Sol® was increased to 5.0 g, now acting as both disintegrant and bioadhesive component. The tablets show a rapid release of ANP and an enhanced uptake of ANP through the oral mucosa in comparison with conventional peroral formulations. The preparation may be used for the treatment of pulmonary edema.

### Example 4. Preparation of rapidly disintegrating tablets for the administration of omeprazole

Rapidly disintegrating tablets with bio/mucoadhesive properties for sublingual administration were prepared according to example 3, each tablet containong 10 mg of omeprazole. The tablets show a rapid release of omeprazole and an enhanced uptake of omeprazole through the oral mucosa, as well as a reduced swallowing of omeprazole in the saliva, in comparison with conventional peroral formulations. The preparation may be used for the treatment of gastroesophageal reflux.

### Example 5. Preparation of an intranasal powder of atrial natriuretic peptide (ANP)

Ordered mixtures with bio/mucoadhesive properties for intranasal administration were prepared according to example 2, each dosed volume of powder mixture containing 0.7 mg of ANP. In contrast to the composition of example 2, no tablets were compressed and subsequently no addition of binder (Avicel® Ph 101), disintegrant (Ac-Di-Sol®) nor lubricant (magnesium stearate) was made. After insufflation into the nasal cavity the powder showed a rapid dissolution of ANP and an enhanced uptake of ANP through the nasal mucosa in comparison with conventional peroral formulations. The preparation may be used for the treatment of pulmonary edema.

In the foregoing specification, the present invention has been described with reference to various examples and preferred embodiments. However, for a person skilled in the art, it is clear that the scope of the invention is not limited to these examples and embodiments, and that further modifications and variations are possible without departing from the inventive idea. The scope of the invention is thus only limited by the appended claims.

### References

Alderborn, G., Pasanen, K., Nyström, C. (1985) Studies on direct compression of tablets. XI. Characterization of particle fragmentation during compaction by permeametry measurements of tablets. Int. J. Pharm. 23: 79-86

Come, S.J., Morrisey, S.M., Woods, R.J. (1974) A method for the quantitative estimation of gastric barrier mucus. J. Physiol. 242: 116P-117P .

Eriksson, M., Nyström, C., Alderborn, G. (1990) Evaluation of a permeametry technique for surface area measurements of coarse particulate materials. Int. J. Pharm. 63: 189-199

Sundell-Bredenberg, S., Nyström, C. (2001) The possibility of achieving an interactive mixture with high dose homogeneity containing an extremely low proportion of a micronised drug. Eur. J. Pharm. Sci. 12: 285-295

Westerberg, M. (1992) Studies on ordered mixtures for fast release and dissolution of drugs with low aqueous solubility. Ph.D. Thesis. Uppsala University, Reprocentralen, HSC, Uppsala, Sweden

## Claims

1. A pharmaceutical composition for the treatment of acute disorders by sublingual or intranasal administration, comprising an essentially water-free, ordered mixture of microparticles of at least one pharmaceutically active agent adhered to the surfaces of carrier particles, said particles being substantially larger than said microparticles and being insoluble or sparingly soluble in water, and a bioadhesion and/or mucoadhesion promoting agent adhered to the surface of the carrier particles, wherein the carrier particles comprise at least one material selected from the group consisting of pharmaceutically acceptable polymers, a pharmaceutically acceptable inorganic salt selected from dicalcium phosphate dihydrate and mixtures or co-processed qualities of these materials.

2. A composition according to claim 1, wherein the microparticles of said active agent or agents have a weight based mean diameter of less than 10 µm.

3. A composition according to claim 1 or 2, wherein the mean sieve diameter of the carrier particles is less than 750 µm, preferably then from 50 to 500 µm.

4. A composition according to any one of claims 1-4, wherein the carrier particles comprise a brittle material which will fragmentize easily when compressed.

5. A composition according to any one of claims 1-4, wherein the carrier particles contain from 0.1 to 40 weight percent of the bio/mucoadhesion promoting agent, preferably then from 2 to 25 weight percent, based on the total composition.

6. A composition according to claim 5, wherein the bio/mucoadhesion promoting agent is selected from the group consisting of cross-linked polymers, acrylic polymers, cellulose derivatives, natural polymers having bio/mucoadhesive properties, and mixtures thereof.

7. A composition according to claim 6, wherein the bio/mucoadhesion promoting agent is selected from the group consisting of cellulose derivatives and comprising hydroxypropylmethyl cellulose, hydroxyethyl cellulose, hydroxypropyl cellulose, sodium carboxymethyl cellulose, methyl cellulose, ethyl hydroxyethyl cellulose, carboxymethyl cellulose and modified cellulose gum; crosscaramellose; modified starch; acrylic polymers comprising carbomer and its derivatives; polyethylene oxide; chitosan; gelatin; sodium alginate; pectin; scleroglucan; xanthan gum; guar gum; poly-co-(methyl vinyl ether-maleic anhydride); and mixtures thereof.

8. A composition according to any one of claims 1-7, further comprising a pharmaceutically acceptable surfactant in a finely dispersed form and intimately mixed with the active agent or agents.

9. A composition according to claim 8, wherein the surfactant is present in an amount from 0.5 to 5 weight percent of the composition, preferably then 0.5 to 3 weight percent.

10. A composition according to claim 8 or 9, wherein the surfactant is selected from the group consisting of sodium lauryl sulfate, polysorbates, bile acid salts and mixtures thereof.

11. A composition according to any one of claims 1-10, wherein the carrier particles comprise at least one of the materials selected from the group consisting of cellulose, cellulose derivatives, starch, starch derivatives, cross-linked polymers based on starch or cellulose, or polyvinylpyrrolidone.

12. A sublingual composition according to any one of claims 1-11, wherein the composition contain at least one pharmaceutical disintegrating agent promoting the dispersion of the carrier particles with the admixed active agent or agents, over the sublingual mucosa.

13. A sublingual composition according to claim 12, wherein the disintegrating agent is selected from the group consisting of cross-linked polyvinylpyrrolidone, carboxymethyl starch, natural starch, microcrystalline cellulose, cellulose gum, and mixtures thereof.

14. A sublingual composition according to claim 12 or 13, wherein the disintegrating agent is present in an amount from 1 to 10 weight percent of the composition.

15. A composition according to any one of claims 1-14, wherein the pharmaceutically active agent is fentanyl or a pharmaceutically acceptable salt thereof.

16. A composition according to any one of claims 1-15, for the treatment of acute disorders by sublingual administration.

17. A composition according to claim 16, for the treatment of acute or breakthrough pain by sublingual administration of fentanyl or a pharmaceutically acceptable salt thereof.

18. A composition according to any one of claims 1-11 or 15, for the treatment of acute disorders by intranasal administration.

19. A composition according to claim 18, for the treatment of acute or breakthrough pain by intranasal administration of fentanyl or a pharmaceutically acceptable salt thereof.

20. The use of an essentially water-free pharmaceutical composition containing an effective amount of at least one pharmaceutically active agent in the form of microparticles adhered to the surfaces of carrier particles, which are substantially larger than said microparticles and are essentially water-insoluble or sparingly water-soluble, and a bioadhesion and/or mucoadhesion promoting agent adhered to the surface of the carrier particles for the manufacture of a medicament for the treatment of acute disorders, wherein to an individual afflicted with said disorder is administered sublingually or intranasally at least one dose unit of that composition.

21. A use according to claim 20, wherein the pharmaceutically active agent is fentanyl or a pharmaceutically acceptable salt thereof.

22. A use according to claim 21, wherein the fentanyl is administered in an amount from 0.025 to 10 mg, preferably then from 0.05 to 2 mg, per dose unit.

23. The use of a composition according to any one of claims 1 to 19 for the manufacture of a medicament for the treatment of acute disorders, wherein to an individual afflicted with said disorder is administered sublingually or intranasally at least one dose unit-of that composition.

## Patentansprüche

1. Pharmazeutische Zusammensetzung zur Behandlung akuter Störungen durch sublinguale oder intranasale Verabreichung, umfassend ein im wesentlichen wasserfreies geordnetes Gemisch aus Mikroteilchen von mindestens einem pharmazeutisch aktiven Mittel, die an den Oberflächen von Trägerteilchen haften, wobei die Teilchen im wesentlichen größer als die Mikroteilchen sind und in Wasser unlöslich oder kaum löslich sind, und einem die Bioadhäsion und/oder Mukoadhäsion fördernden Mittel, das an der Oberfläche der Trägerteilchen haftet, wobei die Trägerteilchen mindestens ein Material, das aus der Gruppe von pharmazeutisch akzeptablen Polymeren, einem pharmazeutisch akzeptablen anorganischen Salz, das aus Dicalciumphosphatdihydrat ausgewählt ist, und Gemischen oder gemeinsam verarbeiteten Qualitäten dieser Materialien ausgewählt ist, umfassen.

2. Zusammensetzung nach Anspruch 1, wobei die Mikroteilchen des aktiven Mittels oder der aktiven Mittel auf Massebasis einen mittleren Durchmesser von weniger als 10 µm aufweisen.

3. Zusammensetzung nach Anspruch 1 oder 2, wobei der mittlere Siebdurchmesser der Trägerteilchen weniger als 750 µm, vorzugsweise dann 50 bis 500 µm beträgt.

4. Zusammensetzung nach einem der Ansprüche 1 bis 4, wobei die Trägerteilchen ein sprödes Material umfassen, das, wenn es zusammengepresst wird, ohne weiteres fragmentiert.

5. Zusammensetzung nach einem der Ansprüche 1 bis 4, wobei die Trägerteilchen 0,1 bis 40 Gew.-% an dem die Bio/Mukoadhäsion fördernden Mittel, vorzugsweise dann 2 bis 25 Gew.-%, bezogen auf die Gesamtzusammensetzung, enthalten.

6. Zusammensetzung nach Anspruch 5, wobei das die Bio/Mukoadhäsion fördernde Mittel aus der Gruppe von vernetzten Polymeren, Acrylpolymeren, Cellulosederivaten, natürlichen Polymeren mit bio/mukoadhäsiven Eigenschaften und Gemischen derselben ausgewählt ist.

7. Zusammensetzung nach Anspruch 6, wobei das die Bio/Mukoadhäsion fördernde Mittel aus der Gruppe von Cellulosederivaten, die Hydroxypropylmethylcellulose, Hydroxyethylcellulose, Hydroxypropylcellulose, Natriumcarboxymethylcellulose, Methylcellulose, Ethylhydroxyethylcellulose, Carboxymethylcellulose und modifizierten Cellulosegummi umfassen; Croscarmellose; modifizierter Stärke; Acrylpolymeren, die Carbomer und dessen Derivate umfassen; Polyethylenoxid; Chitosan; Gelatine; Natriumalginat; Pektin; Skleroglucan; Xanthangummi; Guargummi; Poly-co-(methylvinylether-maleinsäureanhydrid); und Gemischen derselben ausgewählt ist.

8. Zusammensetzung nach einem der Ansprüche 1 bis 7, die ferner ein pharmazeutisch akzeptables grenzflächenaktives Mittel in einer fein dispergierten Form und in innigem Gemisch mit dem aktiven Mittel oder den aktiven Mitteln umfasst.

9. Zusammensetzung nach Anspruch 8, wobei das grenzflächenaktive Mittel in einer Menge von 0,5 bis 5 Gew.-% der Zusammensetzung, vorzugsweise dann 0,5 bis 3 Gew.-% vorhanden ist.

10. Zusammensetzung nach Anspruch 8 oder 9, wobei das grenzflächenaktive Mittel aus der Gruppe von Natriumlaurylsulfat, Polysorbaten, Gallensäuresalzen und Gemischen derselben ausgewählt ist.

11. Zusammensetzung nach einem der Ansprüch 1 bis 10, wobei die Trägerteilchen mindestens eines der Materialien, die aus der Gruppe von Cellulose, Cellulosederivaten, Stärke, Stärkederivaten, vernetzten Polymeren auf der Basis von Stärke oder Cellulose oder Polyvinylpyrrolidon ausgewählt sind, umfassen.

12. Sublinguale Zusammensetzung nach einem der Ansprüche 1 bis 11, wobei die Zusammensetzung mindestens ein pharmazeutisches, den Zerfall förderndes Mittel enthält, das die Dispersion der Trägerteilchen mit dem zugemischten aktiven Mittel oder den zugemischten aktiven Mitteln über die sublinguale Mukosa fördert.

13. Sublinguale Zusammensetzung nach Anspruch 12, wobei das den Zerfall fördernde Mittel aus der Gruppe von vernetztem Polyvinylpyrrolidon, Carboxymethylstärke, natürlicher Stärke, mikrokristalliner Cellulose, Cellulosegummi und Gemischen derselben ausgewählt ist.

14. Sublinguale Zusammensetzung nach Anspruch 12 oder 13, wobei das den Zerfall fördernde Mittel in einer Menge von 1 bis 10 Gew.-% der Zusammensetzung vorhanden ist.

15. Zusammensetzung nach einem der Ansprüche 1 bis 14, wobei das pharmazeutisch aktive Mittel Fentanyl oder ein pharmazeutisch akzeptables Salz desselben ist.

16. Zusammensetzung nach einem der Ansprüche 1 bis 15 zur Behandlung von akuten Störungen durch sublinguale Verabreichung.

17. Zusammensetzung nach Anspruch 16 zur Behandlung von akutem oder Durchbruchschmerz durch sublinguale Verabreichung von Fentanyl oder einem pharmazeutisch akzeptablen Salz desselben.

18. Zusammensetzung nach einem der Ansprüche 1 bis 11 oder 15 zur Behandlung von akuten Störungen durch intranasale Verabreichung.

19. Zusammensetzung nach Anspruch 18 zur Behandlung von akutem oder Durchbruchschmerz durch intranasale Verabreichung von Fentanyl oder einem pharmazeutisch akzeptablen Salz desselben.

20. Verwendung einer im wesentlichen wasserfreien pharmazeutischen Zusammensetzung, die eine wirksame Menge von mindestens einem pharmazeutisch aktiven Mittel in der Form von Mikroteilchen, die an den Oberflächen von Trägerteilchen haften, die im wesentlichen größer als die Mikroteilchen sind und essentiell wasserunlöslich oder kaum wasserlöslich sind, und ein die Bioadhäsion und/oder Mukoadhäsion förderndes Mittel, das an der Oberfläche der Trägerteilchen haftet, enthält, zur Herstellung eines Medikaments zur Behandlung akuter Störungen, wobei einem Individuum, das von der Störung betroffen ist, sublingual oder intranasal mindestens eine Dosiseinheit der Zusammensetzung verabreicht wird.

21. Verwendung nach Anspruch 20, wobei das pharmazeutisch aktive Mittel Fentanyl oder ein pharmazeutisch akzeptables Salz desselben ist.

22. Verwendung nach Anspruch 21, wobei das Fentanyl in einer Menge von 0,025 bis 10 mg, vorzugsweise dann 0,05 bis 2 mg, pro Dosiseinheit verabreicht wird.

23. Verwendung einer Zusammensetzung nach einem der Ansprüche 1 bis 19 zur Herstellung eines Medikaments für die Behandlung akuter Störungen, wobei einem Individuum, das von der Störung betroffen ist, sublingual oder intranasal mindestens eine Dosiseinheit der Zusammensetzung verabreicht wird.

## Revendications

1. Composition pharmaceutique destinée au traitement de troubles aigus par administration sublinguale ou intranasale, comprenant un mélange ordonné et essentiellement dépourvu d'eau de microparticules d'au moins un agent pharmaceutiquement actif collées aux surfaces de particules vectrices, lesdites particules étant sensiblement plus grosses que lesdites microparticules et étant insolubles ou faiblement solubles dans l'eau ; et un agent de promotion de la bioadhésion et/ou de la mucoadhésion collé à la surface des particules vectrices, dans laquelle les particules vectrices comprennent au moins un matériau choisi dans le groupe constitué par les polymères pharmaceutiquement acceptables, un sel inorganique pharmaceutiquement acceptable choisi parmi le phosphate dicalcique dihydrate, et des mélanges ou qualités co-transformées de ces matériaux.

2. Composition selon la revendication 1, dans laquelle les microparticules dudit agent actif ou desdits agents actifs ont un diamètre moyen basé sur le poids inférieur à 10 µm.

3. Composition selon la revendication 1 ou 2, dans laquelle le diamètre de tamis moyen des particules vectrices est inférieur à 750 µm, de préférence de 50 à 500 µm.

4. Composition selon l'une quelconque des revendications 1 à 4, dans laquelle les particules vectrices comprennent un matériau friable qui se fragmentera facilement lorsqu'il sera compressé.

5. Composition selon l'une quelconque des revendications 1 à 4, dans laquelle les particules vectrices contiennent de 0,1 à 40 pour cent en poids de l'agent de promotion de la bio/mucoadhésion, de préférence de 2 à 25 pour cent en poids, par rapport à la composition totale.

6. Composition selon la revendication 5, dans laquelle l'agent de promotion de la bio/mucoadhésion est choisi dans le groupe constitué par les polymères réticulés, les polymères acryliques, les dérivés de la cellulose, les polymères naturels ayant des propriétés de bio/mucoadhésion, et des mélanges de ceux-ci.

7. Composition selon la revendication 6, dans laquelle l'agent de promotion de la bio/mucoadhésion est choisi dans le groupe constitué par les dérivés de la cellulose et comprenant l'hydroxypropyl-méthylcellulose, l'hydroxyéthylcellulose, l'hydroxypropylcellulose, la carboxyméthylcellulose sodique, la méthylcellulose, l'éthylhydroxyéthylcellulose, la carboxyméthylcellulose et la gomme de cellulose modifiée ; la croscarmellose ; l'amidon modifié ; les polymères acryliques comprenant le carbomère et ses dérivés ; l'oxyde de polyéthylène ; le chitosan ; la gélatine ; l'alginate de sodium ; la pectine ; le scléroglucane ; la gomme de xanthane ; la gomme de guar ; le poly-co-(éther méthylvinylique-anhydride maléique) ; et des mélanges de ceux-ci.

8. Composition selon l'une quelconque des revendications 1 à 7, comprenant en outre un surfactant pharmaceutiquement acceptable sous une forme finement dispersée et intimement mélangé à l'agent actif ou aux agents actifs.

9. Composition selon la revendication 8, dans laquelle le surfactant est présent en une quantité de 0,5 à 5 pour cent en poids de la composition, de préférence de 0,5 à 3 pour cent en poids.

10. Composition selon la revendication 8 ou 9, dans laquelle le surfactant est choisi dans le groupe constitué par le lauryl sulfate de sodium, les polysorbates, les sels d'acides biliaires et des mélanges de ceux-ci.

11. Composition selon l'une quelconque des revendications 1 à 10, dans laquelle les particules vectrices comprennent au moins un des matériaux choisis dans le groupe constitué par la cellulose, les dérivés de la cellulose, l'amidon, les dérivés de l'amidon, les polymères réticulés à base d'amidon ou de cellulose, ou la polyvinylpyrrolidone.

12. Composition sublinguale selon l'une quelconque des revendications 1 à 11, dans laquelle la composition contient au moins un agent de désintégration pharmaceutique promouvant la dispersion des particules vectrices avec l'agent actif ou les agents actifs mélangé(s), sur la muqueuse sublinguale.

13. Composition sublinguale selon la revendication 12, dans laquelle l'agent de désintégration est choisi dans le groupe constitué par la polyvinylpyrrolidone réticulée, le carboxyméthyl amidon, l'amidon naturel, la cellulose microcristalline, la gomme de cellulose, et des mélanges de ceux-ci.

14. Composition sublinguale selon la revendication 12 ou 13, dans laquelle l'agent de désintégration est présent en une quantité de 1 à 10 pour cent en poids de la composition.

15. Composition selon l'une quelconque des revendications 1 à 14, dans laquelle l'agent pharmaceutiquement actif est le fentanyl ou un sel pharmaceutiquement acceptable de celui-ci.

16. Composition selon l'une quelconque des revendications 1 à 15, destinée au traitement de troubles aigus par administration sublinguale.

17. Composition selon la revendication 16, destinée au traitement de la douleur aiguë ou d'un accès douloureux paroxystique par administration sublinguale de fentanyl ou d'un sel pharmaceutiquement acceptable de celui-ci.

18. Composition selon l'une quelconque des revendications 1 à 11 ou 15, destinée au traitement de troubles aigus par administration intranasale.

19. Composition selon la revendication 18, destinée au traitement de la douleur aiguë ou d'un accès douloureux paroxystique par administration intranasale de fentanyl ou d'un sel pharmaceutiquement acceptable de celui-ci.

20. Utilisation d'une composition pharmaceutique essentiellement dépourvue d'eau contenant une quantité efficace d'au moins un agent pharmaceutiquement actif sous la forme de microparticules collées aux surfaces de particules vectrices, qui sont sensiblement plus grosses que lesdites microparticules et sont sensiblement insolubles ou faiblement solubles dans l'eau, et un agent de promotion de la bioadhésion et/ou de la mucoadhésion collé à la surface des particules vectrices, pour la fabrication d'un médicament destiné au traitement de troubles aigus, dans laquelle on administre à un individu souffrant dudit trouble au moins une unité de dose de cette composition par voie sublinguale ou intranasale.

21. Utilisation selon la revendication 20, dans laquelle l'agent pharmaceutiquement actif est le fentanyl ou un sel pharmaceutiquement acceptable de celui-ci.

22. Utilisation selon la revendication 21, dans laquelle le fentanyl est administré en une quantité de 0,025 à 10 mg, de préférence de 0,05 à 2 mg, par unité de dose.

23. Utilisation d'une composition selon l'une quelconque des revendications 1 à 19, pour la fabrication d'un médicament destiné au traitement de troubles aigus, dans laquelle on administre à un individu souffrant dudit trouble au moins une unité de dose de cette composition par voie sublinguale ou intranasale.
